# EUROPEAN PATENT SPECIFICATION

(11) **EP 2 310 850 B1**
(45) Date of publication and mention of the grant of the patent: **17.10.2018**
(21) Application number: 09799852.0
(22) Date of filing: 14.07.2009
(51) Int. Cl.: G01N 33/49, G01N 33/50, C12Q 1/68, G01N 33/53, G01N 33/569, C07K 16/28

(54) **A DIAGNOSTIC METHOD**
DIAGNOSEVERFAHREN
PROCÉDÉ DE DIAGNOSTIC

(30) Priority: 25.07.2008 US 83872
(43) Date of publication of application: 20.04.2011
(60) Divisional application: 18193479.5
(73) Proprietor: Cellestis Limited, Carnegie, Victoria 3163 (AU)
(72) Inventor: HOWARD, Jenny, Louise, Bentleigh East Victoria 3165 (AU); RADFORD, Anthony, J., Southbank Victoria 3006 (AU); ROTHEL, James, Stuart, Glen Huntly Victoria 3163 (AU)
(74) Representative: Dehns
(86) International application number: PCT/AU2009/000899
(87) International publication number: WO 2010/009494

(56) References cited:
- WO-A1-2004/042396
- WO-A1-2008/113119
- WO-A2-2006/138478
- DENIS MICHEL ET AL: "Enhancement of the sensitivity of the whole-blood gamma interferon assay for diagnosis of Mycobacterium bovis infections in cattle.", CLINICAL AND VACCINE IMMUNOLOGY : CVI NOV 2007 LNKD- PUBMED:17881504, vol. 14, no. 11, November 2007 (2007-11), pages 1483-1489, XP002669352, ISSN: 1556-6811
- RIBEIRO-RODRIGUES R ET AL: "A role for CD4+CD25+T cells in regulation of the immune response during human tuberculosis", CLINICAL AND EXPERIMENTAL IMMUNOLOGY,, vol. 144, no. 1, 1 January 2006 (2006-01-01), pages 25-34, XP008143588,
- POTT G B ET AL: "Frequency of beryllium-specific, TH1-type cytokine-expressing CD4<+> T cells in patients with beryllium-induced disease", JOURNAL OF ALLERGY AND CLINICAL IMMUNOLOGY, MOSBY, INC, US, vol. 115, no. 5, 1 May 2005 (2005-05-01), pages 1036-1042, XP004875347, ISSN: 0091-6749, DOI: 10.1016/J.JACI.2005.01.019
- ORIOL MANUEL AND DEEPALI KUMAR: "QuantiFERON TM -TB Gold assay for the diagnosis of latent tuberculosis infection", EXPERT REVIEW OF MOLECULAR DIAGNOSTICS, FUTURE DRUGS, LONDON, GB, vol. 8, no. 3, 1 May 2008 (2008-05-01), pages 247-256, XP009156387, ISSN: 1473-7159, DOI: 10.1586/14737159.8.3.247
- RIBEIRO-RODRIGUES R ET AL.: 'A role for CD4+CD25+T cells in regulation of the immune response during human tuberculosis' CLINICAL AND EXPERIMENTAL IMMUNOLOGY vol. 144, no. 1, 2006, pages 25 - 34, XP008143588
- BARIL L ET AL.: 'Pneumococcal surface protein A (PspA) is effective at eliciting T cell mediated response during invasive pneumococcal disease in adults' CLINICAL AND EXPERIMENTAL IMMUNOLOGY vol. 145, no. 2, 2006, pages 277 - 286, XP008143201
- FATOOHI AF ET AL.: 'Cellular Immune Response to Recombinant Antigens in Pregnant Women Chronically Infected with Toxoplasma gondii' CLINICAL AND DIAGNOSTIC LABORATORY IMMUNOLOGY vol. 9, no. 3, 2002, pages 704 - 707, XP002563344
- BENYOUCEF S ET AL.: 'AN INTERFERON-y (IFN- y) BASED WHOLE BLOOD ASSAY TO DETECT T CELL RESPONSE TO ANTIGENS IN HIV-1 INFECTED PATIENTS' PATHOLOGIE BIOLOGIE vol. 45, no. 5, 1997, pages 400 - 403, XP008051763
- B Manning ET AL: "CpG DNA functions as an effective adjuvant for the induction of immune responses in aged mice", Experimental Gerontology, vol. 37, no. 1, 1 December 2001 (2001-12-01), pages 107-126, XP55392465, AMSTERDAM, NL ISSN: 0531-5565, DOI: 10.1016/S0531-5565(01)00157-7

## Description

### FILING DATA

This application is associated with and claims priority from United States Provisional Patent Application No. 61/083,872, filed on 25 July, 2008.

### FIELD

The present invention relates generally to the field of immunological-based diagnostic assays. More particularly, the present invention contemplates an *in vitro* method for measuring cell-mediated immune response reactivity. The present invention further contemplates a cell-mediated immune response-based assay to detect or monitor a disease or condition.

### BACKGROUND

Bibliographic details of the publications referred to by author in this specification are collected alphabetically at the end of the description.

Reference to any prior art in this specification is not, and should not be taken as, an acknowledgment or any form of suggestion that this prior art forms part of the common general knowledge in any country.

Immunological-based diagnostics provides an important tool in detecting a variety of disease conditions. This is especially the case given the specificity of components within the immune system. Notwithstanding this specificity, immunological-based diagnostics are not necessarily always sensitive enough to detect low levels of immune activity, such as in response to a low grade infection or in the presence of a persistent low level infection. There is a need to develop diagnostic assays with enhanced sensitivity.

One form of immunological-based diagnostic assays involve the stimulation of T-cells with antigens or mitogens in either isolated cell culture or in whole blood culture followed by the detection of effector molecules such as cytokines produced by the activated T-cells (also referred to as effector T-cells). The effector molecules are generally detected using techniques such as enzyme immunoassays, multiplex bead analysis, ELISpot and flow cytometry. Such assays are useful for detecting disease-specific T-cell responses. In this regard, see for example WO2004/042396 and Manuel & Kumar, 2008, Expert Reviews of Molecular Diagnostics, Future Drugs, 8(3), p247-256.

In one form of the assay, a T-cell response is measured over a relatively short time period, typically less than 24 hours using whole blood. Such assays are useful in the diagnosis of tuberculosis infection. However, an impediment to these types of assays is the effect of regulatory T-cells (T-reg cells) which suppress the effector T-cell response. Methods have been developed which require the selective depletion of T-reg cells but these are time consuming, reduce the ability for quantitative analysis and reduce sensitivity (see Rodrigues et al., 2006, Clin. Exp. Immunol., 144(254), p25-34). These methods also do not take into account T-cells which augment the immune response. Denis et al., 2007, Clin. Vacc. Immunol., 14(11), p1483-1489 teaches uses of immunomodulators to enhance immune responses in *in vitro* tests. Only antibodies to IL-10 enhanced antigen-specific responses. Manning et al., 2001, Exp. Geront., 37, p107-126 teaches uses of CpG oligonucleotides *in vivo* as adjuvants for vaccination. WO2006/138478 teaches compositions and methods for modulating Toll-like receptors for enhancing survival of activated CD4+ T cells.

An improved immune cell-mediated based assay is needed. Such assays are useful in detecting or monitoring a range of diseases and conditions in a subject and in monitoring therapeutic protocols.

### SUMMARY

The present invention provides a method for detecting cell-mediated immune response activity *via* enhanced effector molecule production. Whilst cell isolation and depletion steps may also be undertaken, the assay of the present invention can be conducted using whole blood without need for laborious cell isolation or depletion steps. The assay of the present invention exhibits enhanced sensitivity. In an embodiment, the effector T-cells are contacted with an antigen associated with a disease or condition to be assessed following or simultaneously with an agent which modulates T-cell and in particular T-reg cell activity or function. The method of the present invention is particularly useful in detecting or monitoring a disease or condition including the level or stage of the disease or condition in a subject such as an infection by a pathogenic agent, an autoimmune disease, cancer and an inflammatory condition. Other conditions include exposure to toxic agents such as beryllium. (Pott et al., 2005, J. Allergy Clin. Immunol., 115(5), p1036-1042 teaches that exposure to beryllium leads to beryllium-specific T-cells.) The assay of the present invention is also useful in monitoring therapeutic protocols.

Accordingly, one aspect of the present invention contemplates an *in vitro* method for measuring cell-mediated immune response activity in a subject, the method comprising contacting a source of T-cells from the subject with an agent which modulates the function or activity of T-cells or a subset thereof, wherein the agent is a sense or antisense oligonucleotide to mRNA or DNA encoding JAK1 or TYK2 which inhibits the function or activity of T-regulatory cells, and an antigen to which the cell-mediated immune response is to be tested, incubating the T-cells with the antigen and agent and measuring the presence or elevation in the level of an immune effector molecule from T-cells wherein the presence or level of the immune effector molecule is indicative of the level of cell-mediated responsiveness of the subject.

Another aspect of the present invention provides an *in vitro* method for detecting the presence, or absence of infection by a pathological agent or exposure to a toxic agent in a subject, the method comprising contacting a source of T-cells from the subject with an agent which modulates the function or activity of T-cells or a subset thereof, wherein the agent is a sense or antisense oligonucleotide to mRNA or DNA encoding JAK1 or TYK2 which inhibits the function or activity of T-regulatory cells, and an antigen to which the cell-mediated immune response is to be tested, incubating the T-cells with the antigen and agent and measuring the presence or elevation in the level of an immune effector molecule from T-cells wherein the presence or level of the immune effector molecule is indicative of infection by a pathogenic agent or exposure to a toxic agent.

Also disclosed herein is an assay to detect the presence, absence, level or stage of a disease or condition in a subject, the method comprising contacting a source of T-cells from the subject with an agent which modulates the function or activity of T-cells or a subset thereof and an antigen to which a cell-mediated immune response is to be tested and measuring the presence or elevation in the level of an immune effector molecule from T-cells wherein the presence or level of the immune effector molecule is indicative of the disease or condition.

Further disclosed herein is a method for monitoring a response to a therapeutic protocol for a disease or condition in a subject, the method comprising contacting a source of T-cells from the subject with an agent used in the therapeutic protocol and measuring the presence or elevation in the level of an immune effector molecule from T-cells wherein the presence or level of the immune effector molecule is indicative of the efficacy of the therapeutic protocol.

The method of the present invention may also be referred to as an "assay". The assay herein is useful *inter alia* in assessing the general immune responsiveness of a subject or for detecting the responsiveness to specific disease conditions such as autoimmune disease, Celiac's disease, cancer or infection by a pathogenic organism or agent. The source of T-cells is conveniently whole blood but the present invention contemplates the use of any source of T-cells including fractionated samples comprising T-cells as well as samples having undergone cell isolation and/or depletion. Optionally, a simple sugar such as dextrose or glucose is added to the reaction mixture. Reference to "whole blood" includes whole blood without dilution as well as where whole blood is used in an assay at a volume of from about 10% to about 100% of the total sample assay volume (i.e. reaction mixture).

In methods disclosed herein, T-reg cells in a T-cell sample are targeted by the agent for function or activity modification. The modification may be to inhibit T-reg cells which have an immune suppressor function or to augment particular cells which have an immune stimulatory function. Examples of agents include CD25 ligands, sense or antisense oligonucleotides to particular genes or mRNA encoding molecules such as a Janus Kinase 1 (JAK1) or a Tyrosine Kinase 2 (TYK2) and stimulating agents such as CpG containing oligonucleotides which act *via* toll-like receptors (TLRs) and/or *via* other mechanisms. Hence, the methods described herein use a CD25 ligand, an oligonucleotide complementary or homologous to genetic material (RNA or DNA) encoding a JAK1 or TYK2 molecule to augment or enhance the sensitivity of an immune cell-mediated assay. The oligonucleotides disclosed herein may have a modified backbone or have chemically modified nucleotides or nucleosides such as phosphorothioates-modified oligonucleotide.

One type of agent may be used or two or all three types of agents may be employed together.

The subjects may be human or non-human animals. Hence, the present invention has human medical, veterinary and livestock applications. Humans represent a particularly useful subject in the practice of the present invention.

Hence, disclosed herein is a method for detecting the presence, absence, level or stage of a disease or condition in a human subject, the method comprising contacting whole blood, which comprises at least 10% of the total volume in a reaction mixture, with an agent which inhibits regulatory T-cell function and an antigen to which a cell-mediated immune response is to be tested and measuring the presence or elevation in the level of an immune effector molecule from T-cells wherein the presence or level of the immune effector molecule is indicative of the disease or condition.

Kits and skin tests are also disclosed herein.

In an embodiment, the sample is whole blood which is collected in collection tubes containing the antigen or to which the antigen is added for incubation. Generally, blood is maintained in the presence of heparin. Heparin may be in the tube when blood is added or is added subsequently. The use of blood collection tubes is compatible with standard automated laboratory systems and these are amenable to analysis in large-scale and random access sampling. Blood collection tubes also minimize handling costs and reduce laboratory exposure to whole blood and plasma and, hence, reduce the risk of laboratory personnel from contracting a pathogenic agent such as human immunodeficiency virus (HIV), hepatitis B virus (HBV) or hepatitis C virus (HCV). Furthermore, use of the collection tubes to conduct the incubation renders the assay more sensitive than the previously used 24 well culture well plates.

The present invention provides an enhanced cell-mediated immune assay, therefore, comprising in one embodiment the use of a collection tube, optionally a simple sugar such as dextrose and the incubation step with an antigen and an agent, wherein the agent is a sense or antisense oligonucleotide to mRNA or DNA encoding JAK1 or TYK2 which modulates T-cell function or activity. The incubation step is generally from about 5 to about 50 hours.

The immune effector molecules are generally a cytokine such as but not limited to IFN-γ or an interleukin (e.g. IL-2, IL-4, IL-6, IL-10, IL-12 or IL13 or transforming growth factor beta [TGFβ] or a granulocyte or granulocyte macrophage colony stimulating factor [G-CSF and GM-CSF, respectively]). The presence or level of immune effector may be determined at the level of the molecule itself or to the extent to which a gene is expressed encoding the molecule.

### DETAILED DESCRIPTION

Throughout this specification, unless the context requires otherwise, the word "comprise", or variations such as "comprises" or "comprising", is to understood to imply the inclusion of a stated element or integer or group of elements or integers but not the exclusion of any other element or integer or group of elements or integers.

Unless otherwise indicated, the subject invention is not limited to specific assay components, biological materials or reagents and the like, as such may vary. It is also to be understood that the terminology used herein is for the purpose of describing particular embodiments only and is not intended to be limiting.

It must be noted that, as used in the subject specification, the singular forms "a", "an" and "the" include plural aspects unless the context clearly dictates otherwise. Thus, for example, reference to "a T-cell" includes a single T-cell, as well as two or more T-cells; reference to "an effector molecule" includes a single effector molecule, as well as two or more effector molecules; reference to "the invention" includes single or multiple aspects of an invention; and so forth.

Terms such as "agent", "reagent", "compound" and "cell" are used herein to refer to a chemical or biological entity which is involved in the assay for detecting a cell mediated response or the level of such a response.

Reference to an "agent", "reagent", "compound" and "cell" also includes combinations of two or more of such entities. A "combination" also includes multi-part such as a two-part composition where the agents are provided separately and used or dispensed separately or admixed together prior to dispensation. For example, a multi-part assay pack may have two or more agents separately maintained. Hence, this aspect of the present invention includes antigens and T-cell modulating agents dried and loose or immobilized to a compartment wall or solid support in an assay pack.

The present invention is predicated in part by the augmentation of production of effector molecules from stimulated T-cells. This enables a more sensitive assay to assess the cell mediated immune responsiveness of a subject. The present invention provides, therefore, an assay to detect, assess or otherwise monitor a cell-mediated response in a subject by measuring the presence or level of effector molecules from effector T-cells stimulated by an antigen of interest, such as an antigen associated with a disease or condition. In the T-cells or a subset of T-cells are contacted with an agent which modulates T-cell function or activity and in particular which modulates T-regulatory cells function or activity. Generally, the agent inhibits the suppressor function of T-reg cells. In accordance with the invention, the agent is a sense or antisense oligonucleotide to mRNA or DNA encoding JAK1 or TYK2 which inhibits the function or activity of T-regulatory cells. The present invention provides, therefore, a means to determine the responsiveness of cell-mediated immune activity in a subject and, in turn, provides a means for the diagnosis of infectious diseases, pathological conditions, level of immunocompetence and a marker of T-cell responsiveness to endogenous or exogenous antigen as well as assessing exposure to a toxic agent such as beryllium.

Accordingly, one aspect of the present invention contemplates an *in vitro* method for measuring cell-mediated immune response activity in a subject, the method comprising contacting a source of T-cells from the subject with an agent which modulates the function or activity of T-cells or a subset thereof, wherein the agent is a sense or antisense oligonucleotide to mRNA or DNA encoding JAK1 or TYK2 which inhibits the function or activity of T-regulatory cells, and an antigen to which the cell-mediated immune response is to be tested, incubating the T-cells with the antigen and agent and measuring the presence or elevation in the level of an immune effector molecule from T-cells wherein the presence or level of the immune effector molecule is indicative of the level of cell-mediated responsiveness of the subject.

Another aspect of the present invention provides an *in vitro* method for detecting the presence, or absence of infection by a pathological agent or exposure to a toxic agent in a subject, the method comprising contacting a source of T-cells from the subject with an agent which modulates the function or activity of T-cells or a subset thereof, wherein the agent is a sense or antisense oligonucleotide to mRNA or DNA encoding JAK1 or TYK2 which inhibits the function or activity of T-regulatory cells, and an antigen to which the cell-mediated immune response is to be tested, incubating the T-cells with the antigen and agent and measuring the presence or elevation in the level of an immune effector molecule from T-cells wherein the presence or level of the immune effector molecule is indicative of infection by a pathogenic agent or exposure to a toxic agent.

Also disclosed herein is an assay to detect the presence, absence, level or stage of a disease or condition in a subject, the method comprising contacting a source of T-cells from the subject with an agent which modulates the function or activity of T-cells or a subset thereof and an antigen to which a cell-mediated immune response is to be tested and measuring the presence or elevation in the level of an immune effector molecule from T-cells wherein the presence or level of the immune effector molecule is indicative of the disease or condition.

Another aspect of the invention provides use of an agent which modulates the function or activity of T-cells or a subset of T-cells, wherein the agent is a sense or antisense oligonucleotide to mRNA or DNA encoding JAK1 or TYK2 which inhibits the function or activity of T-regulatory cell, in a diagnostic assay of cell-mediated immune responsiveness for detecting or monitoring the presence or absence of, infection by a pathogenic agent, and/or exposure to a toxic agent, wherein said diagnostic assay comprises a method of the invention.

The invention also provides a method of identifying a subject having a pathogenic infection, said method comprising contacting a source of T-cells obtained from the subject with an agent which modulates the function or activity of T-cells or a subset thereof, wherein the agent is a sense or antisense oligonucleotide to mRNA or DNA encoding JAK1 or TYK2 which inhibits the function or activity of T-regulatory cells, and an antigen to which the cell-mediated immune response is to be tested and measuring the presence or elevation in the level of an immune effector molecule from T-cells wherein the presence or level of the immune effector molecule is indicative of the level of cell-mediated responsiveness of the subject which is indicative of a pathogenic infection.

Reference to the modulation of T-cell function or activity includes modulating regulatory T-cell (T-reg cells). More particularly, it encompasses inhibiting the suppressor function of T-reg cells. Furthermore, measuring "an immune effector molecule" includes measuring one or more different types of molecules. Agents which modulate T-reg cells include a CD25 ligand; a sense or antisense oligonucleotide to genetic material encoding JAK1 or TYK2; a CpG containing oligonucleotide; an oligonucleotide acting as a TLR modulating agent; and other TLR modulating agents. The methods and uses of the invention use a sense or antisense oligonucleotide to mRNA or DNA encoding JAK1 or TYK2 which inhibits the function or activity of T-regulatory cells.

In a particular embodiment, the T-reg cells are immune response suppressor cells the activity of which is inhibited.

Reference to a "subject" includes a human or non-human species including primates, livestock animals (e.g. sheep, cows, pigs, horses, donkey, goats), laboratory test animals (e.g. mice, rates, rabbits, guinea pigs, hamsters), companion animals (e.g. dogs, cats), avian species (e.g. poultry birds, aviary birds), reptiles and amphibians. The present invention has applicability, therefore, in human medicine as well as having livestock and veterinary and wild-life applications. Most particularly, however, the subject is a human and the cell-mediate immune response assay has applications in screening for responsiveness to pathogenic microorganisms, viruses and parasites, potential for development or monitoring autoimmune conditions, Celiac's disease and for monitoring a subject's response to oncological challenge.

The immune effector molecules may be any of a range of molecules which are produced in response to cell activation or stimulation by an antigen. Although an interferon (IFN) such as IFN-γ is a particularly useful immune effector molecule, others include a range of cytokines such as interleukins (IL), e.g. IL-2, IL-4, IL-6, IL-10, IL-12 or IL-13, tumor necrosis factor alpha (TNF-α), transforming growth factor beta (TGF-β), a colony stimulating factor (CSF) such as granulocyte (G)-CSF or granulocyte macrophage (GM)-CSF amongst many others such as complement or components in the complement pathway.

Examples of inhibitors or modulators of T-reg function include CD25 ligands such as but not limited to a polyclonal or monoclonal antibody to CD25 or an antigen-binding fragment thereof, humanized or deimmunized polyclonal or monoclonal antibodies to CD25 or a recombinant or synthetic form of the polyclonal or monoclonal antibodies. Other examples of agents include sense or antisense nucleic and molecules directed to the mRNA or DNA (i.e. genetic material) encoding Janus Tyrosine Kinase 1 (JAK1) or Tyrosine Kinase 2 (TYK2) or small molecule inhibitors of JAK1 or TYK2 proteins. Reference to "small molecules" includes immunoglobulin new antigen receptors (IgNARs) as described in International Patent Publication No. WO 2005/118629. Yet still further examples of suitable agents stimulating agents such as CpG molecules which act *via* Toll-like receptors (TLRs) and/or other mechanisms. Hence, CpG containing oligonucleotides and an oligonucleotide acting as a TLR modulating agent may be used in methods described herein. However, in methods and uses of the invention the agent is a sense or antisense oligonucleotide to mRNA or DNA encoding JAK1 or TYK2 which inhibits the function or activity of T-regulatory cells.

A single type of agent may be used or two or more types of agents may be employed. For example, the assay may be conducted with a CD25 ligand and a JAK1/TYK2 sense or antisense oligonucleotide; a CD25 ligand and a TLR modulating agent; a JAK1/TYK2 sense or antisense oligonucleotide and a TLR modulating agent; or a CD25 ligand, a JAK1/TYK2 sense or antisense oligonucleotide and a TLR modulating agent. Alternatively, just one type of agent is employed. In another alternative, a CpG comprising oligonucleotide and a TLR modulating agent is used. In methods and uses of the invention a sense or antisense oligonucleotide to mRNA or DNA encoding JAK1 or TYK2 which inhibits the function or activity of T-regulatory cells is used.

The oligonucleotides may be modified such as having a chemically modified backbone such as a phosphorate backbone and/or chemically modified nucleosides or nucleotides.

A "CpG molecule" means an oligonucleotide comprising a CpG sequence or motif. The methods described herein may use any modulator of Toll-like receptor (TLR) function or other aspect of the immune system.

The agents such as CD25 ligand, antisense molecules and CpG molecules may be free standing in a reactive vessel or may be immobilized to a solid support such as a bead or a side or bottom of a reaction vessel. The agent may also be in dried form which is re-constituted prior to or during use. Similarly, the antigen may be free standing or immobilized in a reactive vessel such as to the vessel itself or a bead or other solid support.

In one embodiment, the sample collected from the subject is generally deposited into a blood collection tube. A blood collection tube includes a blood draw tube or other similar vessel. Conveniently, when the sample is whole blood, the blood collection tube is heparinized. Alternatively, heparin is added to the tube after the blood is collected. Notwithstanding that whole blood is particularly contemplated and a most convenient sample, the present invention extends to other samples containing immune cells such as lymph fluid, cerebral fluid, tissue fluid and respiratory fluid including nasal and pulmonary fluid as well as samples having undergone cell depletion. Reference to "whole blood" includes whole blood which has not been diluted such as with tissue culture, medium, reagents, excipients, etc. In one embodiment, the term "whole blood" includes an assay sample (i.e. reaction mixture) comprising at least 10% by volume whole blood. The term "at least 10% by volume" includes blood volumes of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100% by volume of total assay volume of the reaction mixture. Additional agents may be added such as culture media, enzymes, excipients antigen and the like without departing from the sample comprising "whole blood".

The use of blood collection tubes is compatible with standard automated laboratory systems and these are amenable to analysis in large-scale and random access sampling. Blood collection tubes also minimize handling costs and reduce laboratory exposure to whole blood and plasma and, hence, reduce the risk of laboratory personnel from contracting a pathogenic agent such as HIV or hepatitis B virus (HBV) or hepatitis C virus (HCV).

Combining the incubation step with the collection tube is particularly efficacious and enhances the sensitivity of the assay as does the optional feature of incubating the cells in the presence of a sample sugar such as dextrose or glucose.

The cells of the cell-mediated immune system lose the capacity to mount an immune response in whole blood after extended periods following blood draw from the subject, and responses without intervention are often severely reduced or absent 24 hours following blood draw. The reduction of labor and need for specialized plastic ware allows cell-mediated immune stimulation with antigens to be performed at the point of care locations such as physicians' offices, clinics, outpatient facilities and veterinary clinics or on farms. Once antigen stimulation is complete, the requirement for fresh and active cells no longer exists. IFN-γ and other cytokines or immune effector molecules are stable in plasma and, thus, the sample can be stored, or shipped without special conditions or rapid time requirements in a similar fashion to standard serum samples used for other infectious disease or other disease diagnosis.

The incubation step may be from 5 to 50 hours, such as 5 to 40 hours or 8 to 24 hours or a time period in between including 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 hours.

The ability to measure cell-mediated immunity is important for assessing a subject's ability to respond to an infection by an pathogenic agent such as a microorganism or virus or parasite, to mount an autoimmune response such as in autoimmune diabetes or to protect against cancers or other oncological conditions or to detect an inflammatory condition or to detect exposure or sensitivity of a subject to a toxic agent such as beryllium. Consequently, reference to "measuring a cell-mediated immune response in a subject" includes and encompasses immune diagnosis of infectious and autoimmune diseases, a marker for immunocompetence and the detection of T-cell responses to endogenous and/or exogenous antigens (including a measure of the efficacy of a vaccine) as well as a marker for inflammatory diseases, cancer and toxic agents. Importantly, by inhibiting suppressor T-reg cells or stimulating cells which augment the immune system, the assay's sensitivity is enhanced. Hence, low level infections, for example, can now be detected.

Pathogenic or infectious agents include bacteria, parasites and viruses. Examples of bacteria include Gram positive and Gram negative microorganisms such as *Mycobacterium* species, *Staphylococcus* species, *Streptococcus* species, *Escherichia coli, Salmonella* species, *Clostridium* species, *Shigella* species, *Proteus* species, *Bacillus* species, *Hemophilus* species, *Borrelia* species amongst others. *Mycobacterium tuberculosis* is a particularly useful target as well as conditions arising from infection by *M. tuberculosis* such as tuberculosis (TB). Examples of viruses include Hepatitis virus (Hepatitis B virus and Hepatitis C virus), Herpes virus and Human immune deficiency virus (HIV) as well as diseases resulting therefrom. Parasites include *Plasmodium* species, ringworm, liver parasites and the like. Other pathogenic agents include eukaryotic cells such as yeasts and fungi.

Autoimmune diseases contemplated herein for detection include *inter alia* alopecia areata, ankylosing spondylitis, antiphospholipid syndrome, autoimmune Addison's disease multiple sclerosis, autoimmune disease of the adrenal gland, autoimmune hemolytic anemia, autoimmune hepatitis, autoimmune oophoritis and orchitis, Behcet's disease, bullous pemphigoid, cardiomyopathy, celiac sprue-dermatitis, chronic fatigue syndrome (CFIDS), chronic inflammatory demyelinating, chronic inflammatory polyneuropathy, Churg-Strauss syndrome, cicatricial pemphigoid, crest syndrome, cold agglutinin disease, Crohn's disease, dermatitis herpetiformis, discoid lupus, essential mixed cryoglobulinemia, fibromyalgia, glomerulonephritis, Grave's disease, Guillain-Barre, Hashimoto's thyroiditis, idiopathic pulmonary fibrosis, idiopathic thrombocytopenia purpura (ITP), IgA nephropathy, insulin dependent diabetes (Type I), lichen planus, lupus, Meniere's disease, mixed connective tissue disease, multiple sclerosis, myasthenia gravis, myocarditis, pemphigus vulgaris, pernicious anemia, polyarteritis nodosa, polychondritis, polyglancular syndromes, polymyalgia rheumatica, polymyositis and dermatomyositis, primary agammaglobulinemia, primary biliary cirrhosis, psoriasis, Raynaud's phenomenon, Reiter's syndrome, rheumatic fever, rheumatoid arrthritis, sarcoidosis, scleroderma, Sjogren's syndrome, stiff-man syndrome, systemic lupus erythematosus, Takayasu arteritis, temporal arteritis/giant cell arteritis, ulcerative colitis, uveitis, vasculitis and vitiligo.

It is generally important to assess the potential or actual cell-mediated responsiveness in these individuals. The method of the present invention can also be used to detect the presence or absence of these conditions as well as the level or stage of disease process.

Other disease conditions contemplated include inflammatory disease conditions.

Examples of inflammatory disease conditions contemplated by the present invention include but are not limited to those disease and disorders which result in a response of redness, swelling, pain, and a feeling of heat in certain areas that is meant to protect tissues affected by injury or disease. Inflammatory diseases which can be assessed using the methods of the present invention, include, without being limited to, acne, angina, arthritis, aspiration pneumonia, disease, empyema, gastroenteritis, inflammation, intestinal flu, NEC, necrotizing enterocolitis, pelvic inflammatory disease, pharyngitis, PID, pleurisy, raw throat, redness, rubor, sore throat, stomach flu and urinary tract infections, chronic inflammatory demyelinating polyneuropathy, chronic inflammatory demyelinating polyradiculoneuropathy, chronic inflammatory demyelinating polyneuropathy, chronic inflammatory demyelinating polyradiculoneuropathy.

Cancer therapy also is somewhat dependent on cell-mediated immunity. Cancers contemplated herein include: a group of diseases and disorders that are characterized by uncontrolled cellular growth (e.g. formation of tumor) without any differentiation of those cells into specialized and different cells. Such diseases and disorders include ABL1 protooncogene, AIDS related cancers, acoustic neuroma, acute lymphocytic leukaemia, acute myeloid leukaemia, adenocystic carcinoma, adrenocortical cancer, agnogenic myeloid metaplasia, alopecia, alveolar soft-part sarcoma, anal cancer, angiosarcoma, aplastic anaemia, astrocytoma, ataxia-telangiectasia, basal cell carcinoma (skin), bladder cancer, bone cancers, bowel cancer, brain stem glioma, brain and CNS tumors, breast cancer, CNS tumors, carcinoid tumors, cervical cancer, childhood brain tumors, childhood cancer, childhood leukaemia, childhood soft tissue sarcoma, chondrosarcoma, choriocarcinoma, chronic lymphocytic leukaemia, chronic myeloid leukaemia, colorectal cancers, cutaneous T-Cell lymphoma, dermatofibrosarcoma-protuberans, desmoplastic-small-round-cell-tumor, ductal carcinoma, endocrine cancers, endometrial cancer, ependymoma, esophageal cancer, Ewing's sarcoma, extra-hepatic bile duct cancer, eye cancer, eye: melanoma, retinoblastoma, fallopian tube cancer, fanconi anemia, fibrosarcoma, gall bladder cancer, gastric cancer, gastrointestinal cancers, gastrointestinal-carcinoid-tumor, genitourinary cancers, germ cell tumors, gestational-trophoblastic-disease, glioma, gynaecological cancers, hematological malignancies, hairy cell leukaemia, head and neck cancer, hepatocellular cancer, hereditary breast cancer, histiocytosis, Hodgkin's disease, human papillomavirus, hydatidiform mole, hypercalcemia, hypopharynx cancer, intraocular melanoma, islet cell cancer, Kaposi's sarcoma, kidney cancer, Langerhan's-cell-histiocytosis, laryngeal cancer, leiomyosarcoma, leukemia, Li-Fraumeni syndrome, lip cancer, liposarcoma, liver cancer, lung cancer, lymphedema, lymphoma, Hodgkin's lymphoma, non-Hodgkin's lymphoma, male breast cancer, malignant-rhabdoid-tumor-of-kidney, medulloblastoma, melanoma, merkel cell cancer, mesothelioma, metastatic cancer, mouth cancer, multiple endocrine neoplasia, mycosis fungoides, myelodysplastic syndromes, myeloma, myeloproliferative disorders, nasal cancer, nasopharyngeal cancer, nephroblastoma, neuroblastoma, neurofibromatosis, nijmegen breakage syndrome, non-melanoma skin cancer, non-small-cell-lung-cancer-(NSCLC), ocular cancers, oesophageal cancer, oral cavity cancer, oropharynx cancer, osteosarcoma, ostomy ovarian cancer, pancreas cancer, paranasal cancer, parathyroid cancer, parotid gland cancer, penile cancer, peripheral-neuroectodermal-tumors, pituitary cancer, polycythemia vera, prostate cancer, rare-cancers-and-associated-disorders, renal cell carcinoma, retinoblastoma, rhabdomyosarcoma, Rothmund-Thomson syndrome, salivary gland cancer, sarcoma, schwannoma, Sezary syndrome, skin cancer, small cell lung cancer (SCLC), small intestine cancer, soft tissue sarcoma, spinal cord tumors, squamous-cell-carcinoma-(skin), stomach cancer, synovial sarcoma, testicular cancer, thymus cancer, thyroid cancer, transitional-cell-cancer-(bladder), transitional-cell-cancer-(renal-pelvis-/-ureter), trophoblastic cancer, urethral cancer, urinary system cancer, uroplakins, uterine sarcoma, uterus cancer, vaginal cancer, vulva cancer, Waldenstrom's-macroglobulinemia and Wilms' tumor.

Any of a range of antigens may be employed in the assay such as those specific for a particular organism, virus, autoantigen or cancer cell. Alternatively, more general agents may be used to test generic capacity of a cell-mediated immune response and such agents include mitogens. Examples of the latter include purified protein derivative (PPD) from *Mycobacterium tuberculosis* and tetanus toxoid. In general, however, any peptide, polypeptide or protein, carbohydrate, glycoprotein, phospholipid, phosphoprotein or phospholipoprotein or non-protein chemical agent may be used in the assay system as the antigen or mitogen.

As stated above, detection of the immune effector molecules may be made at the protein or nucleic acid levels. Consequently, reference to "presence or level" of the immune effector molecule includes direct and indirect data. For example, high levels of cytokine mRNA are indirect data showing increased levels of the cytokine.

Ligands to the immune effectors are particularly useful in detecting and/or quantitating these molecules. Antibodies to the immune effectors are particularly useful. Techniques for the assays contemplated herein are known in the art and include, for example, radioimmunoassay, sandwich assays, ELISA and ELISpot. Reference to "antibodies" includes parts of antibodies, mammalianized (e.g. humanized) antibodies, deimmunized antibodies, recombinant or synthetic antibodies and hybrid and single chain antibodies. For skin tests, in humans, humanized or deimmunized antibodies are particularly contemplated herein to detect effector molecules.

Both polyclonal and monoclonal antibodies are obtainable by immunization with the immune effector molecules or antigenic fragments thereof and either type is utilizable for immunoassays. The methods of obtaining both types of sera are well known in the art. Polyclonal sera are less preferred but are relatively easily prepared by injection of a suitable laboratory animal with an effective amount of the immune effector, or antigenic part thereof, collecting serum from the animal and isolating specific sera by any of the known immunoadsorbent techniques. Although antibodies produced by this method are utilizable in virtually any type of immunoassay, they are generally less favoured because of the potential heterogeneity of the product.

The use of monoclonal antibodies in an immunoassay is particularly useful because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques which are well known to those who are skilled in the art.

Therefore, also disclosed herein is a method for detecting an immune effector molecule in a sample comprising T-cells from a subject, the method comprising contacting the sample or an aliquot of the sample with an antibody specific for the immune effector molecule or an antigenic fragment thereof for a time and under conditions sufficient for an antibody-effector complex to form, and then detecting the complex wherein the immune effector molecule is generated after incubation of an antigen with T-cells together with an agent which modulates regulatory T-cell function.

A "sample" includes whole blood or a fraction thereof. This method includes micro-arrays and macro-arrays on planar or spherical solid supports. A micro- or macro-array is useful.

A wide range of immunoassay techniques are available as can be seen by reference to U.S. Patent Nos. 4,016,043, 4,424,279 and 4,018,653.

The following is a description of one type of assay. An unlabeled antibody is immobilized on a solid substrate and the sample to be tested for the immune effector molecules (e.g. cytokines) brought into contact with the bound molecule. After a suitable period of incubation, for a period of time sufficient to allow formation of an antibody-immune effector molecule complex, a second antibody specific to the effector molecule, labeled with a reporter molecule capable of producing a detectable signal, is then added and incubated, allowing time sufficient for the formation of another complex of antibody-effector-labeled antibody. Any unreacted material is washed away, and the presence of the effector molecule is determined by observation of a signal produced by the reporter molecule. The results may either be qualitative, by simple observation of the visible signal, or may be quantitated by comparing with a control sample containing known amounts of antigen. This generalized technique is well known to those skilled in the art as would be any of a number of variations.

In these assays, a first antibody having specificity for the instant immune effectors is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, spheres, discs of microplates, or any other surface suitable for conducting an immunoassay. The binding processes are well known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-120 minutes or where more convenient, overnight) and under suitable conditions (e.g. for about 20°C to about 40°C) to allow binding of any subunit present in the antibody. Following the incubation period, the antibody subunit solid phase is washed and dried and incubated with a second antibody specific for a portion of the effector molecule. The second antibody is linked to a reporter molecule which is used to indicate the binding of the second antibody to the effector molecule.

There are many variations to this assay. One particularly useful variation is a simultaneous assay where all or many of the components are admixed substantially simultaneously. Furthermore, binding of an antibody to a cytokine may be determined by binding of a labeled antibody directed to the first mentioned antibody.

By "reporter molecule" as used in the present specification, is meant a molecule which, by its chemical nature, provides an analytically identifiable signal which allows the detection of antigen-bound antibody. Detection may be either qualitative or quantitative. The most commonly used reporter molecules in this type of assay are either enzymes, fluorophores or radionuclide containing molecules (i.e. radioisotopes) and chemiluminescent molecules. Examples of suitable fluorophores are provided in Table 1. In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, beta-galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-antigen complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample. Again, the present invention extends to a substantially simultaneous assay.

Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. The fluorescent labeled antibody is allowed to bind to the first antibody-antigen complex. After washing off the unbound reagent, the remaining tertiary complex is then exposed to the light of the appropriate wavelength the fluorescence observed indicates the presence of the antigen of interest. Immunofluorescene and enzyme immunoassay techniques are both very well established in the art and are particularly preferred for the present method. However, other reporter molecules, such as radioisotope, chemiluminescent or bioluminescent molecules, may also be employed.

There are a range of other detection systems which may be employed including colloidal gold and all such detection systems are encompassed by the present invention.

The present invention also contemplates genetic assays such as involving PCR analysis to detect RNA expression products of a genetic sequence encoding an immune effector.

In one embodiment, PCR is conducted using pairs of primers, one or both of which are generally labeled with the same or a different reporter molecule capable of giving a distinguishable signal. The use of fluorophores is particularly useful in the practice of the present invention. Examples of suitable fluorophores may be selected from the list given in Table 1. Other labels include luminescence and phosphorescence as well as infrared dyes. These dyes or fluorophores may also be used as reporter molecules for antibodies.

**Table 1**

| ***List of suitable fluorophores*** | | |
|---|---|---|
| **Probe** | **Ex¹ (nm)** | **Em² (nm)** |
| **Reactive and conjugated probes** | | |
| Hydroxycoumarin | 325 | 386 |
| Aminocoumarin | 350 | 455 |
| Methoxycoumarin | 360 | 410 |
| Cascade Blue | 375; 400 | 423 |
| Lucifer Yellow | 425 | 528 |
| NBD | 466 | 539 |
| R-Phycoerythrin (PE) | 480; 565 | 578 |
| PE-Cy5 conjugates | 480; 565; 650 | 670 |
| PE-Cy7 conjugates | 480; 565; 743 | 767 |
| APC-Cy7 conjugates | 650; 755 | 767 |
| Red 613 | 480; 565 | 613 |
| Fluorescein | 495 | 519 |
| FluorX | 494 | 520 |
| BODIPY-FL | 503 | 512 |
| TRITC | 547 | 574 |
| X-Rhodamine | 570 | 576 |
| Lissamine Rhodamine B | 570 | 590 |

| **Probe** | **Ex¹ (nm)** | **Em² (nm)** |
|---|---|---|
| PerCP | 490 | 675 |
| Texas Red | 589 | 615 |
| Allophycocyanin (APC) | 650 | 660 |
| TruRed | 490, 675 | 695 |
| Alexa Fluor 350 | 346 | 445 |
| Alexa Fluor 430 | 430 | 545 |
| Alexa Fluor 488 | 494 | 517 |
| Alexa Fluor 532 | 530 | 555 |
| Alexa Fluor 546 | 556 | 573 |
| Alexa Fluor 555 | 556 | 573 |
| Alexa Fluor 568 | 578 | 603 |
| Alexa Fluor 594 | 590 | 617 |
| Alexa Fluor 633 | 621 | 639 |
| Alexa Fluor 647 | 650 | 688 |
| Alexa Fluor 660 | 663 | 690 |
| Alexa Fluor 680 | 679 | 702 |
| Alexa Fluor 700 | 696 | 719 |
| Alexa Fluor 750 | 752 | 779 |
| Cy2 | 489 | 506 |
| Cy3 | (512); 550 | 570; (615) |
| Cy3,5 | 581 | 596; (640) |
| Cy5 | (625); 650 | 670 |
| Cy5,5 | 675 | 694 |
| Cy7 | 743 | 767 |

| **Nucleic acid probes** | | |
|---|---|---|
| Hoeschst 33342 | 343 | 483 |
| DAPI | 345 | 455 |
| Hoechst 33258 | 345 | 478 |
| SYTOX Blue | 431 | 480 |
| Chromomycin A3 | 445 | 575 |
| Mithramycin | 445 | 575 |
| YOYO-1 | 491 | 509 |
| SYTOX Green | 504 | 523 |
| SYTOX Orange | 547 | 570 |
| Ethidium Bromide | 493 | 620 |
| 7-AAD | 546 | 647 |
| Acridine Orange | 503 | 530/640 |
| TOTO-1, TO-PRO-1 | 509 | 533 |
| Thiazole Orange | 510 | 530 |
| Propidium Iodide (PI) | 536 | 617 |
| TOTO-3, TO-PRO-3 | 642 | 661 |
| LDS 751 | 543; *590* | 712; *607* |

| **Fluorescent Proteins** | | |
|---|---|---|
| Y66F | 360 | 508 |
| Y66H | 360 | 442 |
| EBFP | 380 | 440 |
| Wild-type | 396, 475 | 50, 503 |
| GFPuv | 385 | 508 |

| **Probe** | **Ex¹ (nm)** | **Em² (nm)** |
|---|---|---|
| ECFP | 434 | 477 |
| Y66W | 436 | 485 |
| S65A | 471 | 504 |
| S65C | 479 | 507 |
| S65L | 484 | 510 |
| S65T | 488 | 511 |
| EGFP | 489 | 508 |
| EYFP | 514 | 527 |
| DsRed | 558 | 583 |

| **Other probes** | | |
|---|---|---|
| Monochlorobimane | 380 | 461 |
| Calcein | 496 | 517 |

| | | |
|---|---|---|
| ¹ Ex: Peak excitation wavelength (nm) ² Em: Peak emission wavelength (nm) | | |

Any suitable method of analyzing fluorescence emission is encompassed by the present invention. In this regard, the invention contemplates techniques including but not restricted to 2-photon and 3-photon time resolved fluorescence spectroscopy as, for example, disclosed by Lakowicz et al, Biophys. J. 72:567, 1997, fluorescence lifetime imaging as, for example, disclosed by Eriksson et al, Biophys. J. 2:64, 1993 and fluorescence resonance energy transfer as, for example, disclosed by Youvan et al, Biotechnology et elia 3:1-18, 1997.

Luminescence and phosphorescence may result respectively from a suitable luminescent or phosphorescent label as is known in the art. Any optical means of identifying such label may be used in this regard.

Infrared radiation may result from a suitable infrared dye. Exemplary infrared dyes that may be employed in the invention include but are not limited to those disclosed in Lewis et al, Dyes Pigm. 42(2):197, 1999, Tawa et al, Mater. Res. Soc. Symp. Proc.488 [Electrical, Optical and Magnetic Properties of Organic Solid-State Materials IV], 885-890, Daneshvar et al, J. Immunol. Methods 226(1-2):119-128, 1999, Rapaport et al, Appl. Phys. Lett. 74(3):329-331, 1999 and Durig et al, J. Raman Spectrosc. 24(5):281-285, 1993. Any suitable infrared spectroscopic method may be employed to interrogate the infrared dye. For instance, fourier transform infrared spectroscopy as, for example, described by Rahman et al, J. Org. Chem. 63:6196, 1998 may be used in this regard.

Suitably, electromagnetic scattering may result from diffraction, reflection, polarization or refraction of the incident electromagnetic radiation including light and X-rays. Such scattering can be used to quantitate the level of mRNA or level of protein.

Flow cytometry is particularly useful in analyzing fluorophore emission.

As is known in the art, flow cytometry is a high throughput technique which involves rapidly analyzing the physical and chemical characteristics of particles (e.g. labeled mRNA, DNA or proteins) as they pass through the path of one or more laser beams while suspended in a fluid stream. As each particle intercepts the laser beam, the scattered light and fluorescent light emitted by each cell or particle is detected and recorded using any suitable tracking algorithm as, for example, described hereunder.

A modern flow cytometer is able to perform these tasks up to 100,000 cells/particles s⁻¹. Through the use of an optical array of filters and dichroic mirrors, different wavelengths of fluorescent light can be separated and simultaneously detected. In addition, a number of lasers with different excitation wavelengths may be used. Hence, a variety of fluorophores can be used to target and examine, for example, different immune effectors within a sample or immune effectors from multiple subjects.

Suitable flow cytometers which may be used in the methods of the present invention include those which measure five to nine optical parameters (see Table 2) using a single excitation laser, commonly an argon ion air-cooled laser operating at 15 mW on its 488 nm spectral line. More advanced flow cytometers are capable of using multiple excitation lasers such as a HeNe laser (633 nm) or a HeCd laser (325 nm) in addition to the argon ion laser (488 or 514 nm).

**Table 2**

| ***Exemplary optical parameters which may be measured by a flow cytometer.*** | | | |
|---|---|---|---|
| **Parameter** | **Acronym** | **Detection angle form incident laser beam** | **Wavelength (nm)** |
| Forward scattered light | FS | 2-5° | 488* |
| Side scattered light | SS | 90° | 488* |
| "Green" fluorescence | FL1 | 90° | 510-540^{†} |
| "Yellow" fluorescence | FL2 | 90° | 560-580^{†} |
| "Red" fluorescence | FL3 | 90° | >650^{#} |

| | | | |
|---|---|---|---|
| * using a 488 nm excitation laser ^{†} width of bandpass filter ^{#} longpass filter | | | |

For example, Biggs et al, Cytometry 36:36-45, 1999 have constructed an 11-parameter flow cytometer using three excitation lasers and have demonstrated the use of nine distinguishable fluorophores in addition to forward and side scatter measurements for purposes of immunophenotyping (i.e. classifying) particles. The maximum number of parameters commercially available currently is 17: forward scatter, side scatter and three excitation lasers each with five fluorescence detectors. Whether all of the parameters can be adequately used depends heavily on the extinction coefficients, quantum yields and amount of spectral overlap between all fluorophores (Malemed et al, "Flow cytometry and sorting", 2nd Ed., New York, Wiley-Liss, 1990). However, it will be understood that the present invention is not restricted to any particular flow cytometer or any particular set of parameters. In this regard, the invention also contemplates use in place of a conventional flow cytometer, a microfabricated flow cytometer as, for example, disclosed by Fu et al, Nature Biotechnology 17:1109-1111, 1999.

The assay of the present invention may be automated or semi-automated for high throughput screening or for screening for a number of immune effectors from the one subject. The automation is conveniently controlled by computer software.

Also disclosed herein is a computer program product, therefore, for assessing the presence or absence or the level of one or more immune effectors, the product comprising:-
(1) code that receives, as input values, the identity of a reporter molecule associated with a labeled mRNA or antibody:
(2) code that compares said input values with reference values to determine the level of reporter molecules and/or the identity of the molecule to which the reporter molecule is attached; and
(3) a computer readable medium that stores the codes.

Also disclosed herein is a computer for assessing the presence or absence or level of one or more immune effectors, the computer comprises:-
(1) a machine-readable data storage medium comprising a data storage material encoded with machine-readable data, wherein said machine-readable data comprise input values which identify a reporter molecule associated with a labeled mRNA or antibody;
(2) a working memory for storing instructions for processing said machine-readable data;
(3) a central-processing unit coupled to said working memory and to said machine-readable data storage medium, for processing said machine readable data to compare said values to provide an assessment of the identity or level of reporter molecules or of molecules to which they are attached; and
(4) an output hardware coupled to said central processing unit, for receiving the results of the comparison.

One aspect of the present application includes experiments that demonstrate the cell-mediated immune responsiveness of a subject by measuring responsiveness to particular antigens or mitogens. In an embodiment, one or more samples such as a sample of peripheral blood, of enriched white cell fraction of blood or bronchoalveolar lavage may be obtained from a subject having or suspected of development a particular disease (e.g. autoimmune disease, infection to a pathogenic agent or exposure to beryllium) and the immune responsiveness measured by determination of effector molecules from effector T-cells (e.g. CD4⁺ T-cells). the assay is conducted in the presence of an agent which modulates T-cell function such as regulatory T-cell function. In accordance with the invention the agent is a sense or antisense oligonucleotide to mRNA or DNA encoding JAK1 or TYK2 which inhibits the function or activity of T-regulatory cells.

The immunobinding methods include methods for detecting or quantifying the amount of a reactive component in a sample, which methods require the detection or quantitation of any immunecomplexes formed during the binding process. Here, one would obtain a sample suspected of containing a cytokine and contact the sample with an antibody and then detect or quantify the amount of immunecomplexes formed under the specific conditions.

Contacting the chosen biological sample with the antibody under conditions effective and for a period of time sufficient to allow the formation of immunecomplexes (primary immunecomplexes) is generally a matter of adding the composition to the sample and incubating the mixture for a period of time long enough for the antibodies to form immunecomplexes with, i.e. to bind to, any antigens present. After this time, the sample-antibody composition, such as a tissue section, ELISA plate, ELISpot, dot blot or Western blot, will generally be washed to remove any non-specifically bound antibody species, allowing only those antibodies specifically bound within the primary immunecomplexes to be detected.

Disclosed herein is a method for detecting the presence, absence, level or stage of a disease or condition in a human subject, the method comprising contacting whole blood, which comprises at least 10% of the total volume in a reaction mixture, with an agent which inhibits regulatory T-cell function and an antigen to which a cell-mediated immune response is to be tested and measuring the presence or elevation in the level of an immune effector molecule from T-cells wherein the presence or level of the immune effector molecule is indicative of the disease or condition.

Also disclosed herein are kits for use with the methods described above. An immunodetection kit is disclosed. A kit for analysis of a sample from a subject having or suspected of developing a metal or chemically-induced disease is also disclosed. Further, a kit for analysis of a sample from a subject having or suspected of developing a disease is disclosed. In addition, a kit for assessing the cell-mediated immune responsiveness of a subject before or after a disease state has developed is disclosed.

The immunodetection reagents of the kit may take any one of a variety of forms, including those detectable labels that are associated with or linked to the given antibody or antigen, and detectable labels that are associated with or attached to a secondary binding ligand. Exemplary secondary ligands are those secondary antibodies that have binding affinity for the first antibody or antigen, and secondary antibodies that have binding affinity for a human antibody.

Further suitable immunodetection reagents for use in the kits disclosed herein include the two-component reagent that comprises a secondary antibody that has binding affinity for the first antibody or antigen, along with a third antibody that has binding affinity for the second antibody, the third antibody being linked to a detectable label.

The kits may further comprise a suitably aliquoted composition of antigen or effector molecule, whether labeled or unlabeled, as may be used to prepare a standard curve for a detection assay.

The kits may contain antibody-label conjugates either in fully conjugated form, in the form of intermediates, or as separate moieties to be conjugated by the user of the kit. The components of the kits may be packaged either in aqueous media or in lyophilized form.

The container means of any of the kits will generally include at least one vial, test tube, flask, bottle, syringe or other container means, into which the testing agent, the antibody or antigen may be placed, and preferably, suitably aliquoted. Where a second or third binding ligand or additional component is provided, the kit will also generally contain a second, third or other additional container into which this ligand or component may be placed. The kits disclosed herein will also typically include a means for containing the antibody, antigen, and any other reagent containers in close confinement for commercial sale. Such containers may include injection or blow-molded plastic containers into which the desired vials are retained.

Also disclosed herein is a method of treatment of a subject having a pathogenic infection, an autoimmune disorder or cancer or a propensity for developing such a condition or disorder, said method comprising contacting a source of T-cells from the subject with an agent which modulates the function or activity of T-cells or a subset thereof and an antigen to which the cell-mediated immune response is to be tested and measuring the presence or elevation in the level of an immune effector molecule from T-cells wherein the presence or level of the immune effector molecule is indicative of the level of cell-mediated responsiveness of the subject which is indicative of the presence, absence, level or state of the condition or disorder and then treating the condition or disorder.

The present invention is further described by the following non-limiting Examples.

### EXAMPLE 1

### Development of Assay

Heparinized blood samples were collected from consenting volunteers or donors. Blood samples were collected into Vacuette [registered trade mark] tubes (Greiner Bio-one, Germany).

Aliquots of the blood samples were incubated with either *Mycobacterium tuberculosis* purified protein derivative (PPD, Cellestis Limited, Australia), Tetanus toxoid (CSL Limited, Australia), phytohemagglutinin (Cellestis Limited, Australia) or saline control in a number of different sized blood collection tubes as well as the standard 24-well tissue culture plates recommended by the manufacturers of the bovine whole blood IFN-γ test (Bovigam [registered trade mark], CSL Ltd.) or the human Quantiferon [registered trade mark] test (Cellestis Limited, Australia).

In some experiments, dextrose was added at various concentrations to the blood before initiation of incubation.

Antigen stimulated blood samples were incubated for 16 to 24 hours at 37°C, after which plasma was harvested from above the settled blood cells. The amount of IFN-γ present in each plasma sample was then quantified using the Quantiferon-TB [registered trade] ELISA (Cellestis Limited, Australia) as per the manufacturer's instructions. Samples stimulated with the tetanus toxoid antigen and the saline control were also tested using a more sensitive ELISA for IFN-γ (Quantiferon-CMI; Cellestis Limited, Australia) as per the manufacturer's instructions.

ELISA optical density values for IFN-γ standards run on each ELISA plate were used to construct a standard curve from which the amount of IFN-γ present in each of the test plasma samples was converted to IU/mL values.

The samples were also incubated with a CD25 ligand, oligonucleotide directed to genetic material encoding JAK1 or TYK2 or a CpG oligonucleotide. The oligonucleotide may be phosphorothioated.

### COMPARATIVE EXAMPLE 2

### Effects of CD25 antibody

Beads coated with anti-CD25 antibody are mixed with whole blood. There is no prior purification of white blood cells or peripheral blood lymphocytes. Following mixing with whole blood the blood culture is directly incubated for the production of IFN-y without any further processing to separate or deplete the blood of regulatory T-cells. There is no requirement for specific depletion of the regulatory T-cells by magnetic separation or by any other separation technique. The beads are left in the whole blood culture and no specific attempt is made it to separate the regulatory T-cells from the rest of the white blood cells or blood plasma. It is possible that the beads settle into the red cell layer by natural gravitational force as the red cells settle in the blood incubation process, but there is no intervention to make physical separation of the regulatory T-cell from the rest of the incubating and cultured cells. Is also possible that the binding of the beads to the regulatory T-cell is all that is required for the suppression of the T-cell's function.

Table 3 shows the effect of anti-CD25 antibody coated beads on IFN-γ responses to various protein antigens when used in whole blood culture. It is clear that the addition of the beads to the whole blood culture significantly raised the specific response to the antigens used in a titratable manner. This is without any formal separation of the beads from the whole blood culture. The optimum bead concentration that enhances signal without significant background effects can be determined by routine empirical testing.

**Table 3**

| ***IFN-γ response (IU*/*mL) to antigen in whole blood with and without the addition of anti-CD25 magnetic beads*** | | | | | |
|---|---|---|---|---|---|
| | | **Treatment** | | | |
| | | **Control** | **anti-CD25 beads 20µL** | **anti-CD25 beads 5µL** | **anti-CD25 beads 1µL** |
| **Subject 1** | **Nil** | 0.26 | 0.67 | 0.14 | 0.12 |
| | **TB Antigen** | 0.83 | 4.52 | 2.33 | 0.97 |
| **Subject 2** | **Nil** | 0.06 | 0.07 | 0.06 | 0.06 |
| | **Gliadin 10µg/mL** | 0.17 | 3.11 | 0.74 | 0.2 |

### EXAMPLE 3

### Effects of antisense to kinase genetic material

Signals of cell surface receptors are transmitted to effector molecules within the cell *via* a series of modification and amplification molecules including protein kinase molecules. Several of these are significant in the transduction of the signaling molecules derived from regulatory T-cells, including interleukin 10 (IL-10). These kinases may be inhibited in function in pure cell culture by the addition of normal oligonucleotides. Although oligonucleotides are ineffective in whole blood culture the phosphothioate versions can be used in whole blood culture and can be used with diagnostic protein antigens. When used at the appropriate concentration these modified oligonucleotides substantially enhance the IFN-γ signal derived in the whole-blood culture. Other cytokine molecules generated by the activated T-cells will also be enhanced in quantity, including but not limited to molecules such as interleukin 2 (IL-2), interferon inducible protein 10 (IP10), CCX and other cytokines and chemokines.

Table 4 shows the effect of addition of antisense oligonucleotides against genetic material encoding JAK1 and TYK2 kinases. These are phosphothioate oligonucleotides at various concentrations, which are more effective than the natural oligonucleotide version. In this case the added oligonucleotides also raises the specific response to the antigens used without raising any background responses. These experiments are also done in whole blood, and the efficacy of the oligonucleotides in a complex mixture that is whole blood is high.

**Table 4**

| ***IFN-γ response (IU*/*Ml) to antigen in whole blood with and without the addition of oligonucleotides (JAK 1 and TYK2)*** | | | | |
|---|---|---|---|---|
| | | **Treatment** | | |
| | | **Control** | **JAK1 & TYK2 1µM** | **JAK1 & TYK2 10µM** |
| **Subject 1** | **Nil** | 0.26 | 0.11 | 0.39 |
| | **TB Antigen 1ug/Ml** | 0.83 | 0.91 | 9.79 |
| **Subject 2** | **Nil** | 0.06 | 0.06 | 0.09 |
| | **Gliadin 10µg/Ml** | 0.17 | 0.21 | 0.94 |

### EXAMPLE 4

### Oligonucleotide molecules

An alternative method of stimulating T-cell activity in the presence of inhibitory regulatory molecules is to augment the T-cell response *via* additional stimulatory mechanisms that by themselves alone cannot trigger a diagnostic response.

Table 5 shows the effect of addition of oligonucleotides, with a phosphothioate backbone, and also added to whole blood, but in this case the oligonucleotides contain sequences with the CpG motif. These oligonucleotides also raise the specific response to antigens without affecting background responses in any significant manner. The ability of these compounds to assist in raising cytokine signaling that can be used for diagnostic purposes is novel. The use and efficacy in whole blood is also presumably novel and surprising, as it is for the antisense oligonucleotides and the anti-CD25 antibody coated beads.

**Table 5**

| ***IFN-γ response (IU*/*mL) to antigen in whole blood with and without the addition of oligonucleotides (CpG)*** | | | | |
|---|---|---|---|---|
| | | **Treatment** | | |
| | | **Control** | **CpG 1µM** | **CpG 10µM** |
| **Subject 1** | **Nil** | 0.26 | 0.14 | 0.48 |
| | **TB Antigen** | 0.83 | 0.81 | 8.21 |
| **Subject 2** | **Nil** | 0.06 | 0.05 | 0.17 |
| | **Gliadin 10µg/mL** | 0.17 | 0.13 | 1.14 |

Tables 6 and 7 illustrate the use of all these methods in combination.

**Table 6**

| ***IFN-γ response (IU*/*mL) to antigen in whole blood with and without the addition of antisense oligonucleotides individually and in combination*** | | | | | | |
|---|---|---|---|---|---|---|
| **Antigen** | **Contro l** | **JAK1 10µM** | **TYK2 10µM** | **JAK1+TYK 2 10µM each** | **CPG 10µM** | **JAK+TYK+CP G 10µM each** |
| **Nil** | 0.085 | 0.080 | 0.090 | 0.110 | 0.170 | 0.160 |
| **TB Antigen 1µg/mL** | 0.275 | 0.440 | 0.500 | 0.470 | 0.800 | 0.720 |

**Table 7**

| ***IFN-γ response (IU*/*mL) to antigen in whole blood with and without the addition of antisense oligonucleotides and anti-CD25 magnetic beads. Subjects 1 to 5 in this case do not have known tuberculosis infection*** | | | | | | |
|---|---|---|---|---|---|---|
| **Subject** | **Antigen** | **Control** | **JAK1+TYK 2 10µM** | **CPG 10µM** | **BEADS 20µL** | **JAK1 + TYK2 + CPG 10µM each + BEADS 20µL** |
| **1** | **Nil** | 0.03 | 0.04 | 0.04 | 0.03 | 0.05 |
| | **Gliadin** | 0.04 | 0.05 | 0.04 | 0.07 | 0.14 |
| | **TB** | 0.04 | 0.05 | 0.06 | 0.03 | 0.05 |
| **2** | **Nil** | 0.03 | 0.06 | 0.08 | 0.04 | 0.12 |
| | **Gliadin** | 0.05 | 0.05 | 0.10 | 0.13 | 0.17 |
| | **TB** | 0.04 | 0.05 | 0.05 | 0.04 | 0.09 |
| **3** | **Nil** | 0.04 | 0.05 | 0.12 | 0.12 | 0.28 |
| | **Gliadin** | 0.11 | 0.19 | 0.41 | 0.62 | 0.86 |
| | **TB** | 0.07 | 0.08 | 0.24 | 0.15 | 0.21 |
| **4** | **Nil** | 0.05 | 0.05 | 0.09 | 0.64 | 0.98 |
| | **Gliadin** | 0.14 | 0.30 | 0.36 | 3.34 | 4.63 |
| | **TB** | 0.04 | 0.06 | 0.12 | 1.20 | 0.30 |
| **5** | **Nil** | 0.04 | 0.04 | 0.09 | 0.04 | 0.20 |
| | **Gliadin** | 0.17 | 0.09 | 0.18 | 1.34 | 0.76 |
| | **TB** | 0.04 | 0.07 | 0.12 | 0.07 | 0.30 |

### BIBLIOGRAPHY

Biggs et al, Cytometry 36:36-45, 1999
Daneshvar et al, J. Immunol. Methods 226(1-2):119-128, 1999
Durig et al, J. Raman Spectrosc. 24(5):281-285, 1993
Eriksson et al, Biophys. J. 2: 64, 1993
Fu et al, Nature Biotechnology 17:1109-1111, 1999
Lakowicz et al, Biophys. J. 72:567, 1997
Lewis et al, Dyes Pigm. 42(2):197, 1999
Malemed et al, "Flow cytometry and sorting", 2nd Ed., New York, Wiley-Liss, 1990
Rahman et al, J. Org. Chem. 63:6196, 1998
Rapaport et al, Appl. Phys. Lett. 74(3):329-331, 1999
Tawa et al, Mater. Res. Soc. Symp. Proc.488 [Electrical, Optical and Magnetic Properties of Organic Solid-State Materials IV], 885-890
Youvan et al, Biotechnology et elia 3:1-18, 1997

## Claims

1. An *in vitro* method for measuring cell-mediated immune response activity or for detecting the presence, or absence of infection by a pathological agent or exposure to a toxic agent in a subject, said method comprising contacting a source of T-cells from the subject with an agent which modulates the function or activity of T-cells or a subset thereof, wherein the agent is a sense or antisense oligonucleotide to mRNA or DNA encoding JAK1 or TYK2 which inhibits the function or activity of T-regulatory cells, and an antigen to which the cell-mediated immune response is to be tested, incubating the T-cells with the antigen and agent and measuring the presence or elevation in the level of an immune effector molecule from T-cells wherein the presence or level of the immune effector molecule is indicative of the level of cell-mediated responsiveness of the subject, or of infection by a pathological agent or exposure to a toxic agent.

2. The method of Claim 1 wherein the method is for detecting the presence, or absence of infection by a pathological agent or exposure to a toxic agent in a subject.

3. The method of Claim 1 or 2 wherein the subject is a human.

4. The method of any one of Claims 1 to 3 wherein the source of T-cells is whole blood undiluted.

5. The method of any one of Claims 1 to 3 wherein the source of T-cells is whole blood which comprises from about 10% to 100% by volume of the total assay volume of the reaction mixture.

6. The method of Claim 5 wherein the whole blood comprises from about 50% to 100% by volume of the total assay volume of the reaction mixture.

7. The method of Claim 6 wherein the whole blood comprises from about 80% to 100% by volume of the total assay volume of the reaction mixture.

8. The method of any one of Claims 4 to 6 wherein the whole blood is collected in a tube comprising antigen.

9. The method of any one of Claims 4 to 7 wherein the whole blood is collected in a tube comprising heparin.

10. The method of any one of Claims 1 to 9 wherein the oligonucleotide is phosphorothioated.

11. The method of any one of Claims 1 to 10 wherein the immune effector molecule is a cytokine.

12. The method of Claim 11 wherein the cytokine is IFN-γ.

13. The method of any one of Claims 1 to 12 wherein the immune effectors are detected with antibodies specific for same.

14. The method of Claim 13 wherein the immune effectors are detected using ELISA.

15. The method of Claim 14 wherein the immune effectors are detected using ELISpot.

16. The method of any one of Claims 1 to 15 wherein the immune responsiveness is indicative of infection by a pathological agent or exposure to a toxic agent in the subject.

17. The method of any one of Claims 1 to 16 wherein the infection is by a pathogenic agent selected from *Mycobacterium* species, *Staphylococcus* species, *Streptococcus* species, *Borrelia* species, *Escherichia coli, Salmonella* species, *Clostridium* species, *Shigella* species, *Proteus* species, *Bacillus* species, *Hemophilus* species, Hepatitis virus, Herpes virus and Human immune deficiency virus (HIV).

18. The method of Claim 17 wherein the infection is by *Mycobacterium tuberculosis* or tuberculosis (TB).

19. The method of Claim 17 wherein the infection is by a hepatitis virus.

20. The method of any one of Claims 1 to 16 wherein the toxic agent is beryllium.

21. A method of identifying a subject having a pathogenic infection, said method comprising contacting a source of T-cells obtained from the subject with an agent which modulates the function or activity of T-cells or a subset thereof, wherein the agent is a sense or antisense oligonucleotide to mRNA or DNA encoding JAK1 or TYK2 which inhibits the function or activity of T-regulatory cells, and an antigen to which the cell-mediated immune response is to be tested and measuring the presence or elevation in the level of an immune effector molecule from T-cells wherein the presence or level of the immune effector molecule is indicative of the level of cell-mediated responsiveness of the subject which is indicative of a pathogenic infection.

22. Use of an agent which modulates the function or activity of T-cells or a subset of T-cells, wherein the agent is a sense or antisense oligonucleotide to mRNA or DNA encoding JAK1 or TYK2 which inhibits the function or activity of T-regulatory cells, in a diagnostic assay of cell-mediated immune responsiveness for detecting or monitoring the presence or absence of, infection by a pathogenic agent, and/or exposure to a toxic agent, wherein said diagnostic assay comprises a method as defined in any one of Claims 1 to 20.

## Patentansprüche

1. *In-vitro*-Verfahren zum Messen einer zellvermittelten Immunreaktionsaktivität oder zum Detektieren des Vorhandenseins oder Nichtvorhandenseins einer Infektion mit einem Krankheitserreger oder einer Exposition gegenüber einem toxischen MittelMittel in einem Individuum, wobei das Verfahren das In-Kontakt-Bringen einer Quelle von T-Zellen aus dem Individuum mit einem Mittel, das die Funktion oder Aktivität von T-Zellen oder einer Untergruppe davon moduliert, wobei es sich bei dem Mittel um ein Sense- oder Antisense-Oligonucleotid für JAK1 oder TYK2 codierende mRNA oder DNA handelt, das die Funktion oder Aktivität von regulatorischen T-Zellen hemmt, und mit einem Antigen, gegen das die zellvermittelte Immunreaktion getestet werden soll, das Inkubieren der T-Zellen mit dem Antigen und dem Mittel und das Messen des Vorhandenseins oder einer Erhöhung der Menge eines Immuneffektormoleküls von T-Zellen umfasst, wobei das Vorhandensein bzw. die Menge des Immuneffektormoleküls den Grad einer zellvermittelten Reaktivität des Individuums oder der Infektion mit einem Krankheitserreger oder einer Exposition gegenüber einem toxischen Mittel anzeigt.

2. Verfahren nach Anspruch 1, wobei das Verfahren zum Detektieren des Vorhandenseins oder Nichtvorhandenseins einer Infektion mit einem Krankheitserreger oder einer Exposition gegenüber einem toxischen Mittel in einem Individuum dient.

3. Verfahren nach Anspruch 1 oder 2, wobei es sich bei dem Individuum um einen Menschen handelt.

4. Verfahren nach einem der Ansprüche 1 bis 3, wobei es sich bei der Quelle von T-Zellen um unverdünntes Vollblut handelt.

5. Verfahren nach einem der Ansprüche 1 bis 3, wobei es sich bei der Quelle von T-Zellen um Vollblut handelt, das ungefähr 10 Vol.-% bis 100 Vol.-% des Assay-Gesamtvolumens der Reaktionsmischung umfasst.

6. Verfahren nach Anspruch 5, wobei das Vollblut ungefähr 50 Vol.-% bis 100 Vol.-% des Assay-Gesamtvolumens der Reaktionsmischung umfasst.

7. Verfahren nach Anspruch 6, wobei das Vollblut ungefähr 80 Vol.-% bis 100 Vol.-% des Assay-Gesamtvolumens der Reaktionsmischung umfasst.

8. Verfahren nach einem der Ansprüche 4 bis 6, wobei das Vollblut in einem Röhrchen gesammelt wird, das Antigen umfasst.

9. Verfahren nach einem der Ansprüche 4 bis 7, wobei das Vollblut in einem Röhrchen gesammelt wird, das Heparin umfasst.

10. Verfahren nach einem der Ansprüche 1 bis 9, wobei das Oligonucleotid phosphorothioiert ist.

11. Verfahren nach einem der Ansprüche 1 bis 10, wobei es sich bei dem Immuneffektormolekül um ein Cytokin handelt.

12. Verfahren nach Anspruch 11, wobei es sich bei dem Cytokin um IFN-γ handelt.

13. Verfahren nach einem der Ansprüche 1 bis 12, wobei die Immuneffektoren mit Antikörpern detektiert werden, die für diese spezifisch sind.

14. Verfahren nach Anspruch 13, wobei die Immuneffektoren unter Verwendung eines ELISA detektiert werden.

15. Verfahren nach Anspruch 14, wobei die Immuneffektoren unter Verwendung eines ELISpot detektiert werden.

16. Verfahren nach einem der Ansprüche 1 bis 15, wobei die Immunreaktivität eine Infektion mit einem Krankheitserreger oder eine Exposition gegenüber einem toxischen Mittel in dem Individuum anzeigt.

17. Verfahren nach einem der Ansprüche 1 bis 16, wobei es sich bei der Infektion um eine Infektion mit einem Krankheitserreger handelt, der aus *Mycobacterium-*Arten, *Staphylococcus*-Arten, *Streptococcus*-Arten, *Borrelia*-Arten, *Escherichia coli, Salmonella-*Arten, *Clostridium*-Arten, *Shigella*-Arten, *Proteus*-Arten, *Bacillus*-Arten, *Hemophilus-*Arten, Hepatitis-Virus, Herpes-Virus und einem menschlichen Immunschwächevirus (Human Immune Deficiency Virus, HIV) ausgewählt ist.

18. Verfahren nach Anspruch 17, wobei es sich bei der Infektion um eine Infektion mit *Mycobacterium tuberculosis* oder um Tuberkulose (TB) handelt.

19. Verfahren nach Anspruch 17, wobei es sich bei der Infektion um eine Infektion mit einem Hepatitis-Virus handelt.

20. Verfahren nach einem der Ansprüche 1 bis 16, wobei es sich bei dem toxischen Mittel um Beryllium handelt.

21. Verfahren zum Identifizieren eines Individuums mit einer pathogenen Infektion, wobei das Verfahren das In-Kontakt-Bringen einer Quelle von T-Zellen, die aus dem Individuum erhalten wurden, mit einem Mittel, das die Funktion oder Aktivität von T-Zellen oder einer Untergruppe davon moduliert, wobei es sich bei dem Mittel um ein Sense- oder Antisense-Oligonucleotid für JAK1 oder TYK2 codierende mRNA oder DNA handelt, das die Funktion oder Aktivität von regulatorischen T-Zellen hemmt, und mit einem Antigen, gegen das die zellvermittelte Immunreaktion getestet werden soll, und das Messen des Vorhandenseins oder einer Erhöhung der Menge eines Immuneffektormoleküls von T-Zellen umfasst, wobei das Vorhandensein bzw. die Menge des Immuneffektormoleküls den Grad einer zellvermittelten Reaktivität des Individuums anzeigt, der eine pathogene Infektion anzeigt.

22. Verwendung eines Mittel, welches die Funktion oder Aktivität von T-Zellen oder einer Untergruppe von T-Zellen moduliert, wobei es sich bei dem Mittel um ein Sense- oder Antisense-Oligonucleotid für JAK1 oder TYK2 codierende mRNA oder DNA handelt, das die Funktion oder Aktivität von regulatorischen T-Zellen hemmt, in einem diagnostischen Assay der zellvermittelten Immunreaktivität zum Detektieren oder Überwachen des Vorhandenseins oder Nichtvorhandenseins einer Infektion mit einem Krankheitserreger und/oder einer Exposition gegenüber einem toxischen Mittel, wobei der diagnostische Assay ein Verfahren nach der Definition in einem der Ansprüche 1 bis 20 umfasst.

## Revendications

1. Procédé *in vitro* de mesure de l'activité de réponse immunitaire à médiation cellulaire ou de détection de la présence ou de l'absence d'infection par un agent pathogène ou de l'exposition d'un sujet à un agent toxique, ledit procédé comprenant la mise en contact d'une source de lymphocytes T provenant du sujet avec un agent qui module la fonction ou l'activité de lymphocytes T ou d'un sous-ensemble de ceux-ci, dans lequel l'agent est un oligonucléotide sens ou anti-sens en direction de l'ARNm ou l'ADN codant JAK1 ou TYK2 qui inhibe la fonction ou l'activité de lymphocytes T régulateurs, et un antigène par rapport auquel la réponse immunitaire à médiation cellulaire doit être testée, l'incubation des lymphocytes T avec l'antigène et l'agent et la mesure de la présence ou de l'élévation du taux d'une molécule effectrice immunitaire provenant de lymphocytes T, dans lequel la présence ou le niveau de la molécule effectrice immunitaire est une indication du niveau de capacité de réponse à médiation cellulaire du sujet, ou de l'infection par un agent pathogène ou de l'exposition à un agent toxique.

2. Procédé selon la revendication 1, dans lequel ledit procédé est destiné à la détection de la présence ou de l'absence d'infection par un agent pathogène ou de l'exposition d'un sujet à un agent toxique.

3. Procédé selon la revendication 1 ou 2, dans lequel le sujet est un humain.

4. Procédé selon l'une quelconque des revendications 1 à 3, dans lequel la source de lymphocytes T est du sang entier non dilué.

5. Procédé selon l'une quelconque des revendications 1 à 3, dans lequel la source de lymphocytes T est du sang entier qui comprend d'environ 10 % à 100 % en volume du volume d'analyse total du mélange réactionnel.

6. Procédé selon la revendication 5, dans lequel le sang entier comprend d'environ 50 % à 100 % en volume du volume d'analyse total du mélange réactionnel.

7. Procédé selon la revendication 6, dans lequel le sang entier comprend d'environ 80 % à 100 % en volume du volume d'analyse total du mélange réactionnel.

8. Procédé selon l'une quelconque des revendications 4 à 6, dans lequel le sang entier est prélevé dans un tube comprenant un antigène.

9. Procédé selon l'une quelconque des revendications 4 à 7, dans lequel le sang entier est prélevé dans un tube comprenant de l'héparine.

10. Procédé selon l'une quelconque des revendications 1 à 9, dans lequel l'oligonucléotide est phosphorothioaté.

11. Procédé selon l'une quelconque des revendications 1 à 10, dans lequel la molécule effectrice immunitaire est une cytokine.

12. Procédé selon la revendication 11, dans lequel la cytokine est l'IFN-γ.

13. Procédé selon l'une quelconque 1 à 12, dans lequel les effecteurs immunitaires sont détectés avec des anticorps spécifiques pour ceux-ci.

14. Procédé selon la revendication 13, dans lequel les effecteurs immunitaires sont détectés en utilisant ELISA.

15. Procédé selon la revendication 14, dans lequel les effecteurs immunitaires sont détectés en utilisant ELISpot.

16. Procédé selon l'une quelconque des revendications 1 à 15, dans lequel la capacité de réponse immunitaire est une indication d'une infection par un agent pathogène ou d'une exposition d'un sujet à un agent toxique.

17. Procédé selon l'une quelconque des revendications 1 à 16, dans lequel l'infection est une infection par un agent pathogène choisi parmi les espèces *Mycobacterium,* les espèces *Staphylococcus,* les espèces *Streptococcus,* les espèces *Borrelia, Escherichia coli,* les espèces *Salmonella,* les espèces *Clostridium,* les espèces *Shigella,* les espèces *Proteus,* les espèces *Bacillus,* les espèces *Hemophilus,* le virus de l'hépatite, le virus de l'herpès, et le virus de l'immunodéficience humaine (VIH).

18. Procédé selon la revendication 17, dans lequel l'infection est une infection par *Mycobacterium tuberculosis* ou la tuberculose (TB).

19. Procédé selon la revendication 17, dans lequel l'infection est une infection par un virus de l'hépatite.

20. Procédé selon l'une quelconque des revendications 1 à 16, dans lequel l'agent toxique est le beryllium.

21. Procédé d'identification d'un sujet atteint d'une infection pathogène, ledit procédé comprenant la mise en contact d'une source de lymphocytes T obtenue chez le sujet avec un agent qui module la fonction ou l'activité de lymphocytes T ou d'un sous-ensemble de ceux-ci, dans lequel l'agent est un oligonucléotide sens ou anti-sens en direction de l'ARNm ou l'ADN codant JAK1 ou TYK2 qui inhibe la fonction ou l'activité de lymphocytes T régulateurs, et un antigène par rapport auquel la réponse immunitaire à médiation cellulaire doit être testée, et la mesure de la présence ou de l'élévation du niveau de la molécule effectrice immunitaire provenant de lymphocytes T, dans lequel la présence ou le niveau de la molécule effectrice immunitaire est une indication du niveau de capacité de réponse à médiation cellulaire du sujet, qui est une indication d'une infection par un pathogène.

22. Utilisation d'un agent qui module la fonction ou l'activité de lymphocytes T ou d'un sous-ensemble de lymphocytes T, dans laquelle l'agent est un oligonucléotide sens ou anti-sens en direction de l'ARNm ou l'ADN codant JAK1 ou TYK2 qui inhibe la fonction ou l'activité de lymphocytes T régulateurs dans un essai de diagnostic de la capacité de réponse immunitaire à médiation cellulaire pour la détection ou la surveillance de la présence ou de l'absence d'infection par un agent pathogène, et/ou de l'exposition à un agent toxique, dans laquelle ledit essai de diagnostic comprend un procédé tel que défini selon l'une quelconque des revendications 1 à 20.
